# FASCICULE DE BREVET EUROPEEN

(11) **EP 1 771 199 B1**
(45) Date de publication et mention de la délivrance du brevet: **29.12.2010**
(21) Numéro de dépôt: 05776451.6
(22) Date de dépôt: 09.06.2005
(51) Int. Cl.: A61K 38/28, A61K 9/10, A61K 9/51, A61K 47/42

(54) **FORMULATION COLLOIDALE D'INSULINE LONGUE ACTION ET SA PREPARATION**
KOLLOIDALE INSLUINFORMULIERUNG MIT LANGZEITWIRKUNG UND DEREN HERSTELLUNG
LONG-ACTING COLLOIDAL INSULIN FORMULATION AND PREPARATION THEREOF

(30) Priorité: 19.07.2004 FR 0451578
(43) Date de publication de la demande: 11.04.2007
(73) Titulaire: FLAMEL TECHNOLOGIES, 69200 Vénissieux (FR)
(72) Inventeur: CONSTANCIS, Alain, F-69008 Lyon (FR); NICOLAS, Florence, F-69800 Saint-Priest (FR); MEYRUEIX, Rémi, F-69009 Lyon (FR); SOULA, Olivier, F-69330 Meyzieu (FR)
(74) Mandataire: Fleurance, Raphaël
(86) Numéro de dépôt international: PCT/FR2005/050431
(87) Numéro de publication internationale: WO 2006/016078

(56) Documents cités:
- EP-A- 0 734 720
- FR-A- 2 801 226
- US-A1- 2004 038 885
- US-B1- 6 180 141
- CONSTANCIS A ET AL: "Macromolecular colloids of diblock poly(amino acids) that bind insulin" JOURNAL OF COLLOID AND INTERFACE SCIENCE 15 SEP 1999 UNITED STATES, vol. 217, no. 2, 15 septembre 1999 (1999-09-15), pages 357-368, XP002319782 ISSN: 0021-9797

## Description

Le domaine de la présente invention est celui des médicaments à base d'insuline, en particulier des formulations injectables d'insuline, pour le traitement des diabètes de type I et II chez l'homme et l'animal.
La présente invention concerne, plus précisément, des formulations injectables d'insuline formées par des suspensions colloïdales d'insuline destinées à une administration parentérale journalière et capables de maintenir durant tout le nycthémère chez le patient ou l'animal diabétique, une concentration d'insuline sérique voisine de la concentration basale observée chez le sujet sain.
L'invention concerne, également, des procédés de préparation desdites suspensions colloïdales d'insuline.

Les diabétiques sont assujettis à un traitement très contraignant et imparfait, les obligeant à s'injecter de l'insuline plusieurs fois par jour. Outre les injections d'insuline à absorption rapide, destinées à corriger la montée de glycémie après ou au cours d'un repas, il est également nécessaire de maintenir l'insuline sérique au niveau basal jour et nuit afin d'éviter des effets secondaires très néfastes. Cette dernière correction est particulièrement délicate car la nuit, les patients n'ont pas l'opportunité de se traiter et ainsi de rétablir le niveau souhaitable d'insuline.
Les formulations injectables d'insuline doivent être stables, à savoir que insuline qu'elles contiennent ne doit pas se dégrader au stockage. Elle doit par exemple être toujours pleinement efficace au terme d'un stockage de 2 ans à 5°C. Les formulations injectables d'insuline doivent avoir une rhéologie adaptée aux systèmes d'injection couramment utilisés pour l'insuline. Il existe donc un besoin aigu d'une formulation d'insuline longue action, d'une part, injectable au travers de très fines aiguilles et stable. Le problème de la mise au point d'une telle formulation est connu depuis longtemps et a fait l'objet de nombreux travaux.
Au sens du présent exposé, "insuline" désigne aussi bien l'insuline humaine ou une insuline animale ou encore un analogue de l'insuline.
Au sens du présent exposé, une « formulation d'insuline longue action » est une formulation permettant, d'une part, d'éviter, après administration, tout pic d'hypoglycémie néfaste pour le patient et; d'autre part, de maintenir une action hypoglycémiante sur 24 heures au moins.

### ART ANTERIEUR

Il est fait ci-après état d'un certain nombre de propositions techniques antérieures pour des formulations d'insuline longue action.
Ainsi, on connaît par exemple l'insuline humaine longue action NPH. Il s'agit de suspensions partiellement micro-cristallisées de complexes insuline humaine / zinc /protamine (comme décrites par exemple dans US-B-5,834,422), qui permettent de ralentir la libération in vivo de la protéine. Dans ces suspensions, l'insuline est complexée à la protamine et au zinc pour former un précipité partiellement cristallisé. Après injection sous-cutanée, la vitesse de libération de l'insuline est contrôlée par la cinétique de dissolution in vivo de ce précipité et la cinétique de décomplexation de l'insuline. La durée d'action de ce type d'insuline, bien que prolongée par rapport à celle d'une insuline rapide, ne dépasse pas 16 heures et ne couvre donc pas réellement le nycthémère. Par ailleurs, on observe un pic d'hypoglycémie, après administration de cette insuline humaine longue action NPH. Cette dernière n'est donc pas une véritable insuline longue action telle que définie ci-dessus.

Récemment, le brevet US-B-5,656,722 décrit une nouvelle structure de protéine analogue à l'insuline dénommée GLARGINE® incluse dans une formulation.

Une voie totalement différente pour obtenir une forme à libération prolongée de protéine est divulguée dans le brevet US-B- 5 904 936 (EP-B-O 734 720).
La technique proposée ne consiste ni à modifier chimiquement l'insuline, ni encore à complexer l'insuline avec la protamine et le zinc. Il s'agit plutôt d'adsorber l'insuline humaine sur des nanoparticules biocompatibles formées spontanément, par auto-assemblage dans l'eau de polyaminoacides amphiphiles tels que le poly(L-leucine-b-glutamate de sodium) -ci-après dénommé poly(Leu-bloc-Glu). Cet auto-assemblage conduit à une suspension colloïdale de nanoparticules. L'insuline humaine, lorsqu'elle est mise en présence d'une telle suspension colloïdal, s'adsorbe spontanément sur les particules pour former un complexe non covalent insuline/particule. Après injection sous-cutanée, l'insuline humaine se dissocie progressivement du complexe ce qui permet de maintenir sa concentration plasmatique à une valeur voisine de sa valeur basale pendant une durée prolongée. L'avantage de cette approche est de mettre en oeuvre dans un procédé non dénaturant, de l'insuline humaine non modifiée et un polymère biocompatible, sans faire appel à des tensioactifs potentiellement dénaturants.
Il est précisé dans l'exemple 14 du brevet US-B- 5 904 936 que l'insuline humaine s'associe spontanément à des nanoparticules de poly(Leu-bloc-Glu) jusqu'à un taux maximal de 0,65 mg d'insuline humaine pour 10 mg de poly(Leu-bloc-Glu) soit 6,5 % massique. Cette formulation d'insuline longue action injectable selon l'exemple 14 du brevet US-B- 5 904 936 est perfectible s'agissant des points suivants:
- La formulation pourrait gagner en facilité d'injection, en particulier avec des seringues à aiguilles fines 29G, 30G ou 31G. L'inconfort en résultant pour le patient est d'autant plus pénalisant qu'il s'agit d'une injection quotidienne sur plusieurs dizaines d'années.
- La formulation pourrait gagner en stabilité pour retarder encore la dégradation de l'insuline.
- L'injection sous-cutanée de cette formulation chez le cochon peut parfois conduire à la formation d'érythèmes et d'oedèmes marqués, traduisant une tolérance locale susceptible d'être améliorée, ce qui rend cette formulation difficilement compatible avec une application pharmaceutique journalière sur une très longue durée.
- La formulation est difficilement stérilisable par filtration.

Dans son exemple 9, la demande WO-A-01/37809 décrit une formulation d'induline longue action injectable, dont le pH est de 7,4 et qui comprend une suspension de nanoparticules de polymère poly(Leu-bloc-Glu). Cette suspension comprend (par ml de préparation) : 80 UI d'insuline et 56 mg de polymère, soit un rapport massique insuline/poly(Leu-bloc-Glu) de 5%. Administrée chez le chien Beagle à raison de 2 UI/kg, cette suspension donne lieu à une libération prolongée sur 24 heures environ. Cependant, cette formulation présente une stabilité perfectible comme démontrée dans l'exemple 5 infra de la présente demande.

Fort de cette expérience, la demanderesse a redéfini un cahier des charges pour une formulation d'insuline humaine longue action injectable:
1. La formulation gagnerait à être plus aisément injectable au travers d'une aiguille de petit diamètre (e.g. 29G, 30G ou 31G), afin d'améliorer le confort du patient, et ainsi l'observance du traitement.
2. La formulation d'insuline est perfectible s'agissant de sa stabilité notamment à 4°C et à température ambiante, pour ne pas induire des modifications des propriétés de la formulation ni des dégradations de l'insuline humaine.
3. la formulation serait grandement améliorée si elle était dotée d'une excellente tolérance locale, afin d'être compatible avec une injection journalière sur une durée de plusieurs dizaines d'années.
4. La biodisponibilité de l'insuline fournie par une telle formulation gagnerait à être aussi élevée que possible.
5. L'efficacité de la formulation, mesurée, par exemple, par son effet hypoglycémiant, gagnerait à être la plus élevée possible pendant au moins 24 heures, après l'injection.
6. La formulation gagnerait à avoir une rhéologie permettant un remplissage aisé de la seringue par le patient.
7. L'aptitude à la stérilisation par filtration serait un atout déterminant pour la formulation.

Devant cet état de fait, un objectif essentiel de la présente invention est donc de satisfaire pleinement le cahier des charges sus-décrit.
Un autre objectif essentiel de l'invention est de fournir une formulation d'insuline longue action sous forme de suspension colloïdale, qui maintienne un effet hypoglycémiant important s'étendant sur 24 heures au moins, après une seule administration, par exemple par voie sous-cutanée.
Un autre objectif essentiel est de fournir une formulation d'insuline longue action sous forme de suspension colloïdale, qui soit stable et qui n'induise pas de modification de la structure et de la bioactivité de l'insuline.
Un autre objectif essentiel de la présente invention est de fournir une formulation d'insuline longue action aisément injectable au travers d'une aiguille de faible diamètre (par exemple de gauge 29G, 30G ou 31G).
Un autre objectif essentiel de l'invention est de fournir des formulations d'insuline longue action sous forme de suspensions colloïdales permettant un remplissage aisé d'une seringue au travers d'une aiguille de faible diamètre (par exemple de gauge 29G, 30G ou 31G).
Un autre objectif essentiel de l'invention est de fournir des formulations d'insuline longue action sous forme de suspensions colloïdales injectables à faible volume d'injection et concentration élevée en insuline humaine, typiquement 100 IU/ml, et ce sans nuire à l'efficacité thérapeutique et en particulier à la durée de l'effet hypoglycémiant.
Un autre objectif essentiel de la présente invention est de fournir une formulation d'insuline longue action sous forme de suspension colloïdale, présentant une bonne tolérance locale et une toxicité compatibles avec le traitement chronique des diabétiques. Un autre objectif essentiel de l'invention est de fournir une formulation d'insuline longue action sous forme de suspension colloïdale, dans laquelle l'insuline est une insuline humaine non modifiée.
Un autre objectif essentiel de la présente invention est de fournir une formulation d'insuline longue action sous forme de suspension colloïdale comprenant des nanoparticules de poly(Leu-bloc-Glu) et qui soit filtrable sur des filtres de 0,2 µm à des fins de stérilisation.
Un autre objectif essentiel de la présente invention est de fournir une formulation d'insuline longue action sous forme de suspension colloïdale comprenant des nanoparticules de poly(Leu-bloc-Glu) sur lesquelles les protéines sont adsorbées de façon réversible, non covalente et sans dénaturation.
Un autre objectif essentiel de l'invention est de fournir une suspension colloïdale d'insuline sous forme liquide ou sèche, qui conduise à une formulation satisfaisant aux spécifications visées ci-dessus et qui constitue une forme galénique appropriée et convenable pour une administration sous-cutanée.
Un autre objectif essentiel de l'invention est de proposer un procédé de préparation de ces suspensions colloïdales d'insuline longue action, ledit procédé se devant d'être simple à mettre en oeuvre, non dénaturant pour la protéine et devant en outre toujours assurer de façon fiable la reproductibilité des caractéristiques de la formulation.

Les objectifs ci-dessus, parmi d'autres, sont atteints par la présente invention qui concerne, tout d'abord, une formulation injectable d'insuline longue action comprenant une suspension colloïdale, aqueuse et stable de nanoparticles d'au moins un poly(L-leucine-b-L glutamate de sodium) -ci-après dénommé poly(Leu-bloc-Glu), chargées en insuline, **caractérisée en ce que** :
I- le pH de la formulation est tel que :

| | |
|---|---|
| | 5,8 ≤ pH ≤ 7,0 |
| de préférence, | 6,0 ≤ pH ≤ 7,0, |

II- l'osmolarité O (en mOsmol) de la Formulation est telle que :

| | |
|---|---|
| | 250 ≤ O ≤ 800 |
| de préférence. | 250 ≤ O ≤ 600 |
| et plus préférentiellement encore | 270 ≤ O ≤ 400, |

III- la viscosité v (en mPa.s) de la formulation mesurée selon un mode opératoire Mv est faible, à savoir telle que:

| | |
|---|---|
| | v ≤ 40 |
| de préférence | v ≤ 25 |
| et plus préférentiellement encore | v ≤ 20. |

Le fondement inventif de cette nouvelle suspension colloïdale apte à constituer une formulation galénique injectable d'insuline longue action, réside, notamment, dans la combinaison de paramètres I, II & III sélectionnés dans les fenêtres suivantes:
- une fenêtre de pH contribuant à la bonne stabilité de la suspension formant la formulation injectable d'insuline longue action selon l'invention,
- une fenêtre d'osmolarité qui assure notamment une bonne tolérance locale et une bonne aptitude à la filtration stérilisante pour la formulation.
- et une fenêtre de viscosité qui participe à l'instauration dans la formulation de propriétés rhéologiques telles qu'il est possible de remplir aisément une seringue en aspirant la formulation au travers d'une aiguille de faible diamètre (par exemple de gauge 29G, 30G ou 31G).
Un autre atout de cette nouvelle formulation est son coût raisonnable.

Enfin et surtout cette combinaison non évidente des paramètres sélectionnés I. II & III, confère à la formulation des propriétés rhéologiques telles qu'il est possible de l'injecter aisément au travers d'une aiguille de faible diamètre (par exemple de gauge 29G, 30G ou 31G).

Par ailleurs, la formulation selon l'invention possède de préférence au moins l'une (idéalement toutes) des caractéristiques suivantes :
a. L'osmolarité O de la formulation est ajustée à l'aide d'au moins un sel monovalent ou multivalent (par exemple di ou trivalent).
b. La concentration en poly(Leu-bloc-Glu) est inférieure à 60 mg/ml, de préférence comprise entre 10 et 55 mg/ml et, de préférence encore, comprise entre 30 et 55 mg/ml.
c. Les particules du poly(Leu-bloc-Glu) retenu ont un diamètre hydrodynamique moyen Dh exprimé en nanomètres (nm) et mesuré selon un mode opératoire Md, tel que :

| | |
|---|---|
| | 10 ≤ Dh ≤ 150 |
| de préférence, | 20 ≤ Dh ≤ 100. |

d. Le rapport massique insuline/poly(Leu-bloc-Glu), exprimé en %, est tel que :

| | |
|---|---|
| | 3 ≤ insuline/poly(Lcu-bloc-Glu), |
| de préférence, | 5 ≤ insulinelpoly(Leu-bloc-Glu) ≤ 11. |

e. Le taux maximal de chargement Ta des nanoparticules avec l'insuline, exprimé en % de masse d'insuline associée par rapport à la masse de poly(Leu-bloc-Glu) et mesuré selon un mode opératoire Ma, est tel que :

| | |
|---|---|
| | 10 ≤ Ta |
| de préférence, | 10 ≤ Ta ≤ 40 |
| et, plus préférentiellement encore, | 12 ≤ Ta ≤ 25. |

f. L'insuline est une insuline humaine non modifiée.

Finalement, la formulation injectable d'insuline longue action selon l'invention est dotée des propriétés essentielles suivantes:
◆ une bonne aptitude au remplissage d'une seringue par aspiration au travers d'une aiguille de faible diamètre;
◆ une "injectabilité" aisée au travers d'une aiguille de faible diamètre, ce qui améliore considérablement le confort du patient et accroît donc l'observance du traitement;
◆ une excellente stabilité;
◆ une bonne tolérance locale;
◆ une activité hypoglycémiante étendue sur 24 heures après administration chez l'homme d'une dose standard de 0,6 UI/kg;
◆ une aptitude à la filtration stérilisante sur 0,2 micron, grâce à la petite taille des particules.

Cette suspension présente en outre :
- un faible rapport massique polymère/insuline conduisant à un surcoût limité en polymère;
- une gamme de taille de particules qui assure une bonne tolérance locale et une bonne aptitude à la filtration stérilisante; et
- une gamme de concentration en polymère poly(Leu-bloc-Glu) qui permet d'une part de maintenir la durée de l'effet hypoglycémiant sur 24 heures au moins, tout en permettant une injection aisée.

### DESCRIPTION DETAILLEE DE L'INTENTION

La sélection de ces paramètres pour obtenir une suspension colloïdale d'insuline adaptée en tant que formulation médicamenteuse injectable d'insuline longue action, est le fruit d'importantes et longues recherches sur la mesure et la comparaison des activités pharmacocinétiques et pharmacodynamiques de ces formulations d'insuline sur divers modèles animaux ainsi que de nombreux travaux sur "l'injectabilité", la stabilité, la tolérance et la biocompatibilité de cette suspension particulière, ainsi que sur son caractère injectable, et son aptitude à être aspirée au travers d'une aiguille.

La suspension colloïdale stable formant la formulation comporte des nanoparticules structurées submicroniques, constituées par auto assemblage d'un copolymère poly(Leu-bloc-Glu).
Elles sont aptes :
○ à associer (adsorber) spontanément l'insuline, de façon non covalente, en suspension colloïdale, à l'état non dissous et sans dénaturation, pour former un complexe nanoparticule/insuline,
○ et à libérer celle-ci, notamment in vivo, de manière prolongée et/ou retardée. Enfin, elles sont stables en phase aqueuse en l'absence de tensioactif(s).

La suspension colloïdale de nanoparticules selon l'invention résulte d'une sélection particulière parmi celles décrites de manière générale dans le WO-A-01/37809. Cette sélection particulière a été trouvée après de nombreux essais visant à optimiser les exigences contradictoires du cahier des charges mentionné supra.

Les inventeurs ont dû conduire de longs et de nombreux essais pour procéder à la sélection particulière de paramètres permettant de mettre au point une formulation, à savoir une suspension remplissant le cahier des charges susvisé.

L'optimisation de la stabilité de la suspension passe par un ajustement du pH de la suspension entre 5,8 et 7,0, de préférence entre 6,0 et 7,0 et, plus préférentiellement encore inférieur à 7,4. La stabilité dont il est question est, d'une part, une stabilité physicochimique de la suspension colloïdale de nanoparticules de poly(Leu-bloc-Glu) et, d'autre part, une stabilité de l'actif insulinique en termes d'efficacité thérapeutique (contrôle de la glycémie). Avantageusement la formulation reste stable après un stockage de 2 ans à 5 °C, par exemple.

L'aptitude remarquable de la formulation de l'invention à être facilement injectable au travers d'une aiguille de petit diamètre (par exemple gauge 29G, 30G & 31G ou diamètre : 0,15 à 0,4 mm, longueur : 8 à 20 mm) est appréciée notamment au travers de la force à exercer sur le piston de la seringue. Il est par exemple souhaitable que cette force n'excède pas une valeur raisonnable, e.g. de l'ordre de 40 newtons, de préférence de l'ordre de 30 Newtons, et que le débit ne soit pas inférieur ou égal à 1 ml/min. De façon surprenante, la viscosité mesurée à bas .gradient de cisaillement (mesurée selon le mode opératoire Mv décrit ci-après) ne contrôle pas totalement la force nécessaire pour injecter la suspension colloïdale au travers d'une aiguille de faible diamètre. Il est du mérite de la Demanderesse d'avoir montré que la force à appliquer pour l'injection est d'autant plus faible que la concentration en poly(Leu-bloc-Glu) est basse. Idéalement, la concentration en poly(Leu-bloc-Glu) peut être ajustée, conformément à l'invention, afin d'optimiser le compromis entre la durée d'action et le caractère injectable de la suspension. Cet optimum est obtenu pour une concentration en polymère comprise entre 10 et 60 mg/ml, de préférence entre 20 et 55 mg/ml.

La possibilité de remplir aisément la seringue par aspiration de la formulation au travers d'aiguilles de petit diamètre (par exemple gauge 29G, 30G & 31G ou diamètre : 0,15 à 0,4 mm, longueur : 8 à 20 mm) est également une caractéristique visée. Cette opération doit, par exemple, être typiquement réalisée dans un temps ≤ 120 s, de préférence ≤ 60 s et plus préférentiellement encore ≤ 30 s, pour un volume de 500µl.
Il est du mérite des inventeurs d'avoir établi que, contrairement à ce qui a été montré s'agissant de "l'injectabilité" de la formulation formée par une suspension colloïdale de nanoparticules, la capacité de cette formulation permettre un remplissage rapide d'une seringue dépend de façon importante de la viscosité (mesurée selon le mode opératoire Mv décrit infra) de la suspension. Un remplissage rapide, satisfaisant les conditions énoncées ci dessus, est obtenu lorsque la viscosité v (mesurée selon le mode opératoire Mv décrit infra) est ≤ à 40 mPa.s, de préférence ≤ 25 mPa.s et, plus préférentiellement encore ≤ 20mPa.s, voire en pratique ≤ 15 mPa.s, par exemple.

### Mode Opératoire Mv

Conformément à la présente invention, la détermination du paramètre important qu'est la viscosité v (en mPa.s à 20°C) peut être réalisée, par exemple, à 20°C à l'aide d'un rhéomètre ARl000 (TA Instruments) équipé d'une géométrie cône-plan (4 cm, 2°). La viscosité v est mesurée pour un gradient de cisaillement de 10 se.

Un autre mérite des inventeurs est d'avoir trouver un moyen de réduction de la viscosité, par ajustement de l'osmolarité O (en mOsm) de la suspension à une valeur comprise entre 270 et 800, de préférence entre 270 et 600 et plus préférentiellement encore entre 270 et 400.

De préférence, cet ajustement de l'osmolarité est effectué par ajout d'au moins un sel, et plus spécialement d'un ou plusieurs sels monovalents et/ou multivalents (e.g. divalents ou trivalents), plutôt que par ajout de molécules neutres comme le glycérol, le saccharose ou autres molécules polyhydroxylées. Ces sels peuvent être, par exemple, choisis dans les familles suivantes : chlorure de sodium, chlorure de potassium, chlorure de zinc, mono ou dihydrogénophosphate de sodium ou de potassium, chlorure de magnésium ou de calcium, citrate de sodium, sulfate de sodium, sulfate de potassium ou tout autre sel connu de l'homme de l'art et apte à être injecté par voie sous cutanée.

Suivant une caractéristique remarquable de l'invention, les sels multivalents sont des candidats privilégiés pour l'ajustement de l'osmolarité, étant donné qu'ils ont un effet fluidifiant plus marqué que les sels monovalents.

Avantageusement, ces sels multivalents sont par exemple sélectionnés dans le groupe comprenant: le chlorure de zinc, le chlorure de magnésium, le chlorure de calcium, le phosphate disodique, le citrate de sodium, le citrate de potassium, le sulfate de sodium ou le sulfate de potassium et leurs mélanges.

Conformément à l'invention, les nanoparticules ont une petite taille et présentent plus précisément un diamètre hydrodynamique Dh, mesuré selon le mode opératoire Md, tel que (en nm) dans l'ordre croissant de préférence : 10 ≤ Dh ≤ 150; 10 ≤ Dh ≤100; 20 ≤ Dh ≤ 100; 10 ≤ Dh ≤ 50, 15 ≤ Dh ≤ 40.
L'une des incidences, parmi d'autres, de ces choix de taille de particules de poly(Leu-bloc-Glu) est que la formulation selon l'invention est aisément filtrable sur un filtre stérilisant de 0,2 µm de taille de pores, ce qui permet d'obtenir, aisément et à moindre coût, une formulation injectable stérile. Par ailleurs, il apparaît que de façon surprenante, la tolérance locale de ces particules est meilleure que celle de particules de plus grande taille, comme démontré ci-après dans les exemples.

### Mode opératoire Md :

La poudre pulvérulente de poly(Leu-bloc-Glu) est mise en suspension dans l'eau à une concentration d'environ 50 g/l et est laissée à agiter une nuit à 25°C. Elle est ensuite diluée à l'aide d'une solution aqueuse de chlorure de sodium 0,15 M, de façon à obtenir au final une concentration en poly(Leu-bloc-Glu) comprise entre 0,01 et 0,5 g/l et, de préférence, égale à 0,2 g/l. Cette suspension est agitée 1 heure, puis introduite dans la cellule de diffusion d'un appareil de diffusion de la lumière, de type Brookhaven, fonctionnant avec un faisceau laser de longueur d'onde 488 nm et polarisé verticalement. Le diamètre hydrodynamique est calculé à partir de la fonction d'autocorrélation du champ électrique par le méthode des cumulants, comme décrit dans l'ouvrage « Surfactant Science Series » volume 22, Surfactant Solutions, Ed. R. Zana, chap. 3, M. Dekker, 1984.

Suivant une autre caractéristique préférée, les poly(Leu-bloc-Glu) sélectionnés conformément à l'invention, présentent la caractéristique suivante :
le taux maximal de chargement Ta des nanoparticules avec l'insuline, exprimé en % de masse d'insuline associée par rapport à la masse poly(Leu-bloc-Glu) et mesuré selon un mode opératoire Ma, est tel que : 10 ≤ Ta

| | |
|---|---|
| de préférence, | 10 ≤ Ta ≤ 40 |
| et, plus préférentiellement encore, | 12 ≤ Ta ≤ 25. |

### Mode opératoire Ma :

(a) Préparation de solutions aqueuses d'insuline : de l'insuline recombinante humaine lyophilisée est versée dans un volume V de solution d'acide chlorhydrique 0.01 N durant au maximum 15 min. Cette solution est ensuite versée dans un même volume V de solution NaOH 0.01 N. Le pH est ajusté entre 7.2 et 7.4 avec une solution de soude 1N. La solution est agitée doucement pendant 30 min. La masse d'insuline et le volume V sont calculés en fonction du volume V' souhaité de solution finale afin d'obtenir des concentrations en insuline de 100 UI/ml, 120 UI/ml et 140 UI/ml.
(b) Préparation de la formulation d'insuline. Le poly(Leu-bloc-Glu) lyophilisé est ajouté aux solutions d'insuline à raison de 11 mg/ml. Ces mélanges sont dégazés puis placés dans un agitateur à basculement à 25°C pendant 2 heures puis dégazés de nouveau. Le pH est ajusté entre 7.2 et 7.4 avec une solution HCl 1N et les mélanges sont agités (agitateur à basculement) pendant une nuit à température ambiante.
(c) Dosage de l'insuline libre : les formulations sont injectées sur une colonne de chromatographie liquide d'exclusion stérique dans des conditions non dissociantes et l'insuline libre est dosée par fluorimétrie.

Suivant une variante avantageuse, l'insuline associée aux nanoparticules dans la suspension constituant la formulation de l'invention est une insuline non modifiée.
Au sens de l'invention, une insuline non modifiée est une insuline recombinante ou non, n'ayant subi aucune transformation de sa structure primaire, ni aucune modification des groupements latéraux des acides aminés.

Avantageusement, la suspension constituant la formulation de l'invention comprend au moins un conservateur, de préférence sélectionné dans le groupe comprenant : les phénols, les crésols (e.g méta-crésol), le parahydroxybenzoate de méthyle, de propyle, de butyle, ou tout autre conservateur connu de l'homme de l'art (consulter par exemple l'article de L.A. Gatlin et al. in Injectable Drug Development, P.K.Gupta eds Interpharm Press, Denver Colorado, 1999), et leurs mélanges.

Le procédé de synthèse du poly(Leu-bloc-Glu) ainsi que le procédé d'obtention des nanoparticules de poly(Leu-bloc-Glu) en suspension aqueuse s'effectuent, de préférence, selon les modalités et les recommandations décrites dans le WO-A-01/37809.

Au lieu d'être en suspension stable en milieu liquide aqueux, les nanoparticules de poly(Leu-bloc-Glu) chargées en insuline pourraient également exister dans un état solide stable, de préférence sous forme pulvérulente. Ainsi, la présente invention concerne également un solide -de préférence pulvérulent-, **caractérisé en ce qu**'il comprend des nanoparticules de poly(Leu-bloc-Glu) chargées en insuline et en ce qu'il est obtenu à partir de la suspension liquide qui est définie ci-dessus et qui constitue la formulation de l'invention. Cette obtention est effectuée par tout moyen connu et approprié tel que la lyophilisation, l'atomisation ou le séchage.

Le procédé de préparation de la formulation liquide injectable d'insuline longue action fait intervenir du poly(Leu-bloc-Glu) non chargé en insuline et consiste essentiellement à mettre en suspension, en milieu liquide aqueux, au moins un poly(Leu-bloc-Glu) et de l'insuline, de préférence sous agitation, à ajouter éventuellement des excipients, au besoin à ajuster le pH à une valeur comprise entre 5,8 et 7,0 et, éventuellement à filtrer sur des pores de taille 0,2 µm la suspension ainsi obtenue.

Pour effectuer l'association de l'insuline aux nanoparticules, il est possible de mettre en oeuvre plusieurs méthodes, dont des exemples non limitatifs sont donnés ci-après.
Selon une première méthode, on effectue l'association de l'insuline aux particules par mise en présence d'une phase aqueuse contenant l'insuline avec la suspension colloïdale de nanoparticules de poly(Leu-bloc-Glu). De façon plus précise : une suspension de nanoparticules à pH neutre et isotonique est reconstituée à une concentration de 60 mg/ml ou plus (selon la concentration voulue dans la suspension finale). Une solution d'insuline concentrée (typiquement entre 500 et 600 UI/ml - pH compris entre 7 et 8 - isotonique) est alors préparée extemporanément à partir de poudre d'insuline (dissolution en milieu acide puis neutralisation). Les deux solutions sont mélangées par agitation pendant quelques minutes et cette phase est éventuellement suivie d'une phase de « maturation » de quelques heures. Le pH est ensuite ajusté à une valeur comprise entre 5,8 et 7,0.
Selon une deuxième méthode, il s'agit essentiellement de mettre en présence (en les mélangeant) le poly(Leu-bloc-Glu) à l'état pulvérulent avec une phase aqueuse contenant l'insuline e.g. à une concentration entre 100 et 200 UI/ml.

Il importe de veiller au respect de l'une des caractéristiques essentielles de l'invention, à savoir l'établissement d'un pH compris entre 5,8 et 7,0, de préférence entre 6,0 et 7.0. Ce facteur participe de façon importante à la stabilité et à la tolérance locale de la suspension de nanoparticules de poly(Leu-bloc-Glu). L'ajustement du pH peut s'effectuer par tout moyen connu et approprié, à savoir notamment par acidification, par exemple, de la façon suivante :
- 1- ajout d'acide chlorhydrique 0,1N dans la suspension chargée d'insuline (il se produit alors une précipitation intermédiaire qui disparaît au bout d'une heure d'agitation environ)
- 2- ajout d'acide acétique 0,1N dans la suspension chargée d'insuline (pas de précipitation intermédiaire)
L'ajout -2- d'acide acétique est préféré.

Lors de la préparation de la formulation selon l'invention, on peut éventuellement ajouter des excipients, et au besoin réajuster le pH à une valeur comprise entre 5,8 et 7,0, et, éventuellement stériliser la suspension ainsi obtenue par filtration sur des pores de 0,2 microns.

Ces autres excipients peuvent être notamment au moins un conservateur, de préférence sélectionné dans le groupe comprenant : les phénols, les crésols (e.g méta-crésol), les parahydroxybenzoates de méthyle, de propyle, ou de butyle, ou tout autre conservateur connu de l'homme de l'art (consulter par exemple l'article de L.A. Gatlin et aL in Injectable Drug Development, P.K.Gupta eds Interpharm Press, Denver Colorado, 1999), et leurs mélanges.

Les conditions de mélange, aussi bien pour la régulation du pH que pour l'addition d'excipients, sont classiques et à la portée de l'homme du métier notamment en termes de température, pression et agitation.

Finalement, la suspension liquide peut être transformée en un solide pulvérulent par toute méthode classique connue de l'homme de l'art, telle que par exemple la lyophilisation, l'aton-dsation ou le séchage.

Selon un autre de ses aspects, l'invention concerne les applications pharmaceutiques et vétérinaires de la suspension poly(Leu-bloc-Glu)-insuline. L'application essentielle est le traitement du diabète, et plus précisément du diabète de type 1 et II.

Ainsi, l'invention a également pour objet un médicament **caractérisé en ce qu**'il comprend de la formulation comprenant une suspension colloïdale poly(Leu-bloc-Glu)-insuline, cette formulation étant telle que définie ci-dessus et/ou de la formulation obtenue par le procédé lui aussi décrit supra et/ou du solide pulvérulent tel que défini ci-dessus.

Il s'agit de préférence d'un médicament destiné au traitement du diabète et plus précisément du diabète de type I et II.

Suivant une disposition avantageuse de l'invention, le médicament qu'elle concerne est assimilable à une formulation liquide injectable d'insuline humaine longue action susceptible d'assurer au patient diabétique, après une injection sous-cutanée, une concentration basale en insuline, pendant au moins 24 heures, en régime d'injections répétées.

La concentration basale d'insuline doit s'entendre dans le présent exposé comme une concentration typique dans le sang observée chez l'individu sain, soit 30 picomoles/l.

L'invention concerne en outre une méthode de traitement du diabète, en particulier du diabète de type I et II, **caractérisée en ce qu**'elle consiste essentiellement à administrer au patient le médicament susvisé à base d'une formulation comprenant une suspension colloïdale aqueuse stable de nanoparticules de poly(Leu-bloc-Glu) chargées en insuline, de préférence non modifiée.

Il s'agit préférentiellement d'une administration journalière par injection de préférence sous-cutanée.

Le médicament selon l'invention peut également se présenter sous la forme d'un solide pulvérulent décrit ci-avant et éventuellement du liquide aqueux pour la préparation de la suspension.
Il en résulte que l'invention couvre également une présentation galénique comprenant, d'une part, du solide pulvérulent tel que défini supra et, d'autre part, de manière séparée, du liquide aqueux pour la reconstitution, avant l'administration, de la suspension constituant la formulation selon l'invention.

Les exemples qui suivent permettront de mieux comprendre l'invention dans ses différents aspects produit/procédé/application. Ces exemples illustrent la préparation de la formulation selon l'invention à base de suspension de nanoparticules de poly(Leu-bloc-Glu) chargées en insuline, de même qu'ils présentent les caractéristiques de structure et les propriétés de cette formulation, en la comparant avec des formulations poly(Leu-bloc-Glu)-insuline selon l'art antérieur.

### EXEMPLES

### Exemple 1 - Synthèse d'un poly(Leu-bloc-Glu) 25/35 dibloc

Dans un réacteur de 0,5 litre thermostaté à 30°C, on introduit sous agitation 38,9 g de NCA-GluOMe (0,208 mole) et 156 g de N-Méthyl Pyrrolidine-onc-2 (NMP). Après dissolution, on ajoute 5,78 g d'une solution d'ammoniac 0,452 M dans le méthanol (1,25 % molaire/NCA). La polymérisation est suivie par mesure de dioxyde de carbone dégagé dans une cloche à gaz et vérifiée par disparition des bandes de vibration caractéristiques des NCA à 1860 et 1790 cm-1. Après 30 min, on introduit une solution de 23,3 g de NCA Leucine (0,149 mole) dans 5 219 g de NMP. Après 10 min de réaction, la température est augmentée à 60°C. La polymérisation est suivie comme précédemment. Elle est complète après 1-2 heures. La température du mélange réactionnel obtenu précédemment est augmentée à 80°C. 42,0 g d'acide chlorhydrique aqueux (35 % de la masse) sont ajoutés au milieu réactionnel sous agitation mécanique en 30 min. Le réacteur est alors mis sous pression réduite régulée à 600 mbar pendant 6 heures. Un mélange de 42,0 g d'acide chlorhydrique 35 % et de 167,9 g d'eau est alors ajouté sur 60 min, suivi d'une deuxième phase de vide à 250 mbar pendant 18 heures. Le mélange réactionnel est ensuite refroidi à 50°C, puis neutralisé par la soude-aqueuse (35% de la masse). La NMP et le chloruré de sodium formé lors de la neutralisation, sont éliminés par diafiltration contre 20 volumes d'eau Milli Q, sur membrane de MWCO de 1000 Daltons (système Pellicon II, Millipore). On obtient une suspension colloïdale aqueuse stable de nanoparticules de vectorisation. La suspension de nanoparticules est finalement lyophilisée.
Les teneurs en motifs leucine sont déterminées par résonance magnétique nucléaire du proton (signaux à 2,10, 2,22 et 2,58 ppm pour 4H du Glu et à 0,85 ppm pour 6H du Leu).

### Exemple 2 - Synthèse d'un poly(Leu-bloc-Glu) 25/70 dibloc

146,4 g de NCA GluOMe sont dissous dans 586 g de NMP auxquels sont ajoutés 18,43 g d'une solution d'ammoniac 0,48 M dans le méthanol. Lorsque la polymérisation des NCA GluOMe est complète, une solution de 43,9 g de NCA Leu dans 708 g de NMP est introduite et la polymérisation des NCA Leu est poursuivie jusqu'à disparition des monomères. Le milieu est alors porté à 80°C et on y ajoute, en goutte à goutte, durant 30 min à 1 heure, 129,4 g d'HCl 35 %. Un vide de 600 mbar est appliqué pendant 6 heures, puis 129,4 g d'HCl 35 % supplémentaires sont ajoutés en mélange avec 517,5 g d'eau. Un vide de 250 mbar est alors appliqué pendant 18 heures. Après cette étape, la température est réduite à 50°C, 1 litre d'eau est introduit, suivi de 280 ml de NaOH 35 % pour ramener le pH à 7,4. La suspension est ensuite filtrée (5 µm), dialysée (seuil de coupure 1 000 Da) dans de l'eau, pour éliminer le solvant et les sels, et enfin filtrée (0,22 µm). Cette suspension peut être utilisée directement ou subir des traitements ultérieurs, tels que la distillation de l'eau ou la lyophilisation

### Exemple 3 - Préparation d'une suspension d'insuline longue action selon l'invention

### 3.1- préparation d'une suspension colloïdale de nanoparticules de poly(L leucine bloc L glutamate de sodium) (P) intermédiaire concentrée à 60 mg/g :

La préparation de la suspension est faite sous hotte à flux laminaire ou en pièce stérile. Dans un flacon de verre de 3 l, on introduit successivement 50 g de polymère poly(Leu-bloc-Glu) selon l'exemple 2 sous forme lyophilisée (contenant 1 % d'eau environ) et 757 g d'eau PPI (préparation pour injectable). La suspension est agitée de manière énergique à l'aide d'un barreau magnétique pendant au moins 12 heures. Un vide limité (50-100 mbar) est réalisé dans le flacon. 10,6 g de NaCl 30% et 6,7 g de NaOH 1N sont ajoutés à la solution pour ajuster le pH à 7,2 et l'osmolarité à 300 mOsm et régler la concentration en polymère à 60 mg/ml.

### 3.2- préparation d'une solution d'insuline intermédiaire à 590 UI/ml :

4,5 g d'insuline humaine recombinante (poudre) d'activité 28,4 UI/g et contenant 7,7% d'humidité sont introduits dans un flacon en verre, 181 g d'eau sont rajoutés et l'insuline est dispersée sous agitation magnétique lente. 4,0 g d'HCl 1N sont ajoutés jusqu'à obtention d'une solution limpide d'insuline acide. 5,6 g de soude 1N sont alors ajoutés de façon à obtenir une solution finale ayant un pH compris entre 7 et 8. 6,1 g de NaCl 30% sont ajoutés pour ajuster l'osmolarité.
La solution est filtrée sur membrane polyéthersulfone 0,2µm avant mélange avec la suspension de NPs.

### 3.3 - préparation d'une solution d'excipients (phénol 140 mM ; m-crésol 140 mM, acide acétique 0,1N) :

Dans un flacon de 1 l on rajoute successivement :
   - 13,2 g de phénol (M=94 g/mol)
   - 100 g d'eau
   - 15,1 g de m-crésol (M=108 g/mol)
   - 100 g d'acide acétique 1N
   - 771,7 g d'eau.

La solution est agitée au moins une demi-heure jusqu'à obtention d'une solution limpide puis filtrée sur membrane 0.2 µm PVDF ou PTFE.

### 3.4- préparation d'une suspension colloïdale d'insuline longue action A :

(Osmolarité O = 300 mOsm; pH=6.5; Concentration en poly(Leu-bloc-Glu) Cpol = 42 mg/ml; Concentration en insuline C insulin = 3.5 mg/ml)
800 g de suspension P sont introduits dans un flacon de 1 litre.
Sous agitation magnétique lente, 196 g de solution à 590 UI/ml d'insuline sont rajoutés. L'agitation est maintenue pendant ¼ heure puis le mélange est laissé au repos à 25°C pendant une nuit. 171 g de solution d'excipients sont alors rajoutés au mélange et la solution est laissée à agiter pendant 4 heures. La formulation ainsi obtenue est filtrée sur membrane polyéthersulfone 0,2 µm.

### Exemple 4 - Viscosités et diamètres hydrodynamiques de la suspension d'insuline longue action A selon l'invention

Les mesures de viscosité sont réalisées à 20°C sur un rhéomètre AR1000 (TA Instruments) équipé d'une géométrie cône-plan (4cm, 2°). La viscosité est mesurée pour un gradient de cisaillement de 10 s⁻¹.
On trouve une viscosité de 25 mPa.s.
Le diamètre hydrodynamique des particules des suspensions H, M et L , mesuré selon le mode opératoire Md., est de 35 nm.

### Exemple 5 - Stabilités comparées des suspensions d'insuline longue action à différents pH

Une première suspension pharmaceutique M d'insuline, contenant :
- 100 UI/ml d'insuline recombinante humaine
- 42 mg/ml du polymère poly(Leu-bloc-Glu) selon l'exemple 1
- 21 mM de phénol et de méta-crésol
est préparée selon l'exemple 3 ci-dessus. Son pH est ajusté à pH 6,5 +-0,1 et son osmolarité est de 300+/-20 mOsmol.

Une deuxième suspension pharmaceutique comparative, Comp1, d'insuline longue action, identique à la composition M est préparée de la même façon. Mais à la différence de la suspension selon l'invention, son pH est ajusté à 7,2 +/-0,1. L'osmolarité O est également de 300+/-20 mOsmol.
Les suspensions pharmaceutiques M et Comp1 sont mises en stabilité accélérée à 37°C. Le suivi de stabilité est effectué en mesurant en fonction du temps le pH, l'osmolarité, le diamètre hydrodynamique des nanoparticules chargées en insuline et la quantité d'insuline non dénaturée dans chacune des préparations A et B.

Afin de doser l'insuline dans les préparations M et Comp1, 0,1 ml de préparation sont dissous dans 2 ml d'acide trifluoroacétique (TFA). La solution est ensuite injectée dans une colonne C8 Biobasic® pour analyse HPLC de la protéine. On parvient ainsi à doser l'insuline non dénaturée dans la préparation. La péremption de la préparation est atteinte lorsque le taux d'insuline non dénaturée descend en dessous de 95%.

Pour les deux préparations M et Comp1, le pH, l'osmolarité et le diamètre des nanoparticules sont stables comme le montre le tableau ci-dessous :

Le tableau 1 ci-après montre l'évolution du pH, de l'osmolarité et du diamètre hydrodynamique des particules et du % d'insuline humaine non dénaturée pour les formulations M (pH 6,5) et Comp 1 (pH 7,4).

**TABLEAU 1**

| Temps(jour) | 0 | 7 | 21 |
|---|---|---|---|
| **pH** | | | |
| **M** | 6,5 | 6,3 | 6,5 |
| **Comp1** | 7,2 | 7,3 | 7,2 |

| **Osmolarité O (mOsmol)** | | | |
|---|---|---|---|
| **M** | 303 | 301 | 305 |
| **Comp1** | 297 | 302 | 301 |

| **Diamètre hydrodynamique** (nm) | | | |
|---|---|---|---|
| **M** | 36 | 34 | 35 |
| **Comp1** | 35 | 35 | 34 |

| **% insuline non dénaturée** | | | |
|---|---|---|---|
| **M** | 100 | 98 | 96 |
| **Comp1** | 100 | 95 | 86 |

On constate que la suspension M selon l'invention maintenue à pH 6,5 présente, dans ces conditions de test accéléré, une excellente stabilité puisque 95% de l'insuline est encore non dénaturée après 21 jours. En revanche, pour la formulation Comp1 à pH 7,2, la quantité d'insuline non dénaturée descend au-dessous de 95% dès t = 7 jours. Ainsi, de façon surprenante, la stabilité de l'insuline dans les préparations d'insuline longue action selon la présente invention, est remarquablement bonne à pH légèrement acide.

### Exemple 6 - Comparaison de la tolérance locale d'une suspension d'insuline longue action selon l'invention et d'une suspension d'insuline longue action à base d'un poly(Leu-bloc-Glu) selon WO-A-01/37809 et n'appartenant pas à la sélection selon l'invention

Une étude a été réalisée de manière à comparer la tolérance locale de la suspension H selon l'invention, correspondant à l'exemple 3 ci-dessus et d'une formulation à pH neutre d'un poly(Leu-bloc-Glu) n'appartenant pas à la sélection selon l'invention.

La suspension M selon l'invention comprend:
- 100 UI/ml d'insuline recombinante humaine
- 42 mg/ml du polymère poly(Leu-bloc-Glu) selon l'exemple 3
- 21 mM de phénol et de métacrésol
et est préparée selon l'exemple 3 ci-dessus. Son pH est ajusté à pH 6,5 +-0,1.

Une suspension Comp2 comparative, n'appartenant pas à l'invention, est préparée à partir d'un copolyaminoacide poly(Leu-bloc-Glu) selon le WO-A-01/37809. Elle comprend :
- 100 UI/ml d'insuline recombinante humaine
- 100 mg/ml d'un poly(L leucine bloc L glutamate de sodium) formé de 40 unités de leucine et de 60 unités de glutamate de sodium. Le diamètre hydrodynamique des particules est de 80 nm.
Cette formulation Comp2 est préparée selon le mode opératoire décrit dans l'exemple 3 ci-dessus. Son pH est ajusté à pH 7,4 +-0,1.

Les suspensions M et Comp2 ci-dessus et une solution de NaCl 0.9 % (référence négative) ont été injectées en sous-cutané, sous un volume de 0,50 ml, sur la peau du ventre de 9 porcs domestiques.
Les signes cliniques - érythème et oedème - ont été évalués durant les trois jours qui suivent l'injection.
La référence négative (NaCl) n'a pas provoqué de réaction clinique.

La préparation Comp2 a provoqué une réaction locale allant en augmentant jusqu'à 24 heures. A ce stade, elle se caractérise par un érythème et un oedème cotés modéré à marqué. La réaction disparaît en 3 jours environ.

La préparation M a induit une réaction locale très légère et transitoire. Quelques animaux présentent un érythème très léger à 12 et 24 heures, et un très léger oedème est généralement observé jusqu'à 24 heures.

Ainsi, la tolérance locale de la suspension M selon l'invention est notablement meilleure que celle de la suspension Comp2.

Contrairement à Comp2, la suspension M selon l'invention permet donc l'administration quotidienne du médicament.

### Exemple 7 - Temps de remplissage d'une formulation A selon l'invention

Le temps de remplissage d'une seringue au travers d'une aiguille de gauge 29 et de longueur 8 mm est mesuré pour des valeurs croissantes de l'osmolarité.
La suspension selon l'invention est préparée selon l'exemple 3. Son pH est de 6,5 et sa concentration de 43 mg/ml.
Le volume de remplissage est de 0,5 ml.
L'osmolarité O de la suspension est réglée par ajouts dosés de NaCl.
On obtient les résultats suivants présentés dans le tableau 2 ci-dessous:

**TABLEAU 2**

| Osmolarité O (mOsmol) | Temps de remplissage (s) |
|---|---|
| 120 | Remplissage impossible |
| 200 | 180 |
| 300 | 90 |
| 600 | 50 |

Le réglage de l'osmolarité O selon l'invention permet ainsi de ramener le temps de remplissage d'une seringue à un temps suffisamment court pour permettre aisément un usage quotidien par les patients.

## Revendications

1. Formulation injectable d'insuline longue action comprenant une suspension colloïdale, aqueuse et stable de nanoparticles d'au moins un poly(L-leucine-b-L glutamate de sodium) -ci-après dénommé poly(Leu-bloc-Glu), chargées en insuline,
**caractérisée en ce que** :
I- le pH de la formulation est tel que :
| | |
|---|---|
| | 5,8 ≤ pH ≤ 7,0 |
| de préférence, | 6,0 ≤ pH ≤ 7,0, |
II- l'osmolarité O (en mOsmol) de la formulation est telle que :
| | |
|---|---|
| | 270 ≤ O ≤ 800 |
| de préférence, | 270 ≤ O ≤ 600 |
| et plus préférentiellement encore | 270 ≤ O ≤ 400, |
III - la viscosité v (en mPa.s à 20°C) de la formulation, mesurée à 20°C à l'aide d'un rhéomètre AR1000 (TA Instruments), équipé d'une géométrie cône-plan (4 cm, 2°), pour un gradient de cisaillement de 10 s⁻¹, est faible, à savoir telle que:
| | |
|---|---|
| | v ≤ 40 |
| de préférence | v ≤ 25 |
| et plus préférentiellement encore | v ≤ 20. |

2. Formulation selon la revendication 1, **caractérisée en ce que** son osmolarité O est ajustée à l'aide d'au moins un sel monovalent ou multivalent.

3. Formulation selon la revendication 1 ou 2, **caractérisée en ce que** la concentration en poly(Leu-bloc-Glu) est inférieure à 60 mg/ml, de préférence comprise entre 10 et 55 mg/ml et de préférence encore comprise entre 30 et 55 mg/ml.

4. Formulation selon l'une des revendications précédentes, **caractérisée en ce que** les particules du poly(Leu-bloc-Glu) retenu ont un diamètre hydrodynamique moyen Dh exprimé en nanomètres (nm) et mesuré selon un mode opératoire Md, tel que :
| | |
|---|---|
| | 10 ≤ Dh ≤ 150 |
| de préférence, | 20 ≤ Dh ≤ 100, |
ledit mode opératoire Md étant le suivant : la poudre pulvérulente de poly(Leu-bloc-Glu) est mise en suspension dans l'eau à une concentration d'environ 50 g/l et est laissée à agiter une nuit à 25°C. Elle est ensuite diluée à l'aide d'une solution aqueuse de chlorure de sodium 0,15 M, de façon à obtenir au final une concentration en poly(Leu-bloc-Glu) comprise entre 0,01 et 0,5 g/l et, de préférence, égale à 0,2 g/l. Cette suspension est agitée 1 heure, puis introduite dans la cellule de diffusion d'un appareil de diffusion de la lumière, de type Brookhaven, fonctionnant avec un faisceau laser de longueur d'onde 488 nm et polarisé verticalement.

5. Formulation selon l'une des revendications précédentes, **caractérisée en ce que** le rapport massique insuline/poly(Leu-bloc-Glu), exprimé en %, est tel que :
| | |
|---|---|
| | 3 ≤ insuline/poly(Leu-bloc-Glu), |
| de préférence, | 5 ≤ insuline/poly(Leu-bloc-Glu) ≤ 11. |

6. Formulation selon l'une des revendications précédentes, **caractérisée en ce que** le taux maximal de chargement Ta des nanoparticules avec l'insuline, exprimé en % de masse d'insuline associée par rapport à la masse de poly(Leu-bloc-Glu) et mesuré selon un mode opératoire Ma, est tel que :
| | |
|---|---|
| | 10 ≤ Ta |
| de préférence, | 10 ≤ Ta ≤ 40 |
| et, plus préférentiellement encore, | 12 ≤ Ta ≤ 25, |
ledit mode opératoire Ma étant le suivant:
(a) Préparation de solutions aqueuses d'insuline: de l'insuline recombinante humaine lyophilisée est versée dans un volume V de solution d'acide chlorhydrique 0.01 N durant au maximum 15 min; la solution obtenue est ensuite versée dans un même volume V de solution NaOH 0.01 N ; le pH est ajusté entre 7.2 et 7.4 avec une solution de soude 1 N ; puis la solution est agitée doucement pendant 30 min ;
(b) Préparation de la formulation d'insuline : le poly(Leu-bloc-Glu) lyophilisé est ajouté aux solutions d'insuline préparées en (a) à raison de 11 mg/ml ; les mélanges obtenus sont dégazés puis placés dans un agitateur à basculement à 25°C pendant 2 heures puis dégazés de nouveau ; le pH est ajusté entre 7.2 et 7.4 avec une solution HCl 1N ; les mélanges obtenus sont agités dans un agitateur à basculement pendant une nuit à température ambiante ;
(c) Dosage de l'insuline libre : les formulations obtenues en (b) sont injectées sur une colonne de chromatographie liquide d'exclusion stérique dans des conditions non dissociantes, et l'insuline libre est dosée par fluorimétrie.

7. Formulation selon l'une des revendications précédentes, **caractérisée en ce que** l'insuline est une insuline humaine non modifiée.

8. Formulation selon l'une des revendications précédentes, **caractérisée en ce qu'**elle comprend au moins un conservateur, de préférence sélectionné dans le groupe comprenant : les phénols, les crésols (e.g méta-crésol), les parahydroxybenzoates de méthyle, de propyle, ou de butyle et leurs mélanges.

9. Solide pulvérulent **caractérisé en ce qu'**il comprend des nanoparticules de poly(Leu-bloc-Glu) chargées en insuline et **en ce qu'**il est obtenu à partir de la formulation selon l'une quelconque des revendications 1 à 8.

10. Procédé de préparation de la formulation selon l'une quelconque des revendications 1 à 8, **caractérisé en ce qu'**il consiste essentiellement à mélanger, en milieu liquide aqueux, au moins un poly(Leu-bloc-Glu) et de l'insuline, de préférence sous agitation, à ajouter éventuellement des excipients, au besoin à ajuster le pH à une valeur comprise entre 5,8 et 7,0 et, éventuellement à filtrer la suspension ainsi obtenue.

11. Procédé de préparation de la formulation selon l'une quelconque des revendications 1 à 8, **caractérisé en ce qu'**il consiste essentiellement à mélanger le poly(Leu-bloc-Glu) à l'état pulvérulent, avec une phase aqueuse contenant l'insuline.

12. Procédé de préparation de la formulation selon l'une quelconque des revendications 1 à 8, **caractérisé en ce qu'**il consiste essentiellement à mettre en suspension le solide pulvérulent selon la revendication 8 dans un milieu liquide aqueux, de préférence sous agitation, à ajouter éventuellement des excipients, au besoin à ajuster le pH à une valeur comprise entre 5,8 et 7,0 et éventuellement à filtrer la suspension ainsi obtenue.

13. Médicament **caractérisé en ce qu'**il comprend de la formulation injectable d'insuline longue action selon l'une quelconque des revendications 1 à 8 et/ou de la formulation obtenue par le procédé selon l'une quelconque des revendications 10 à 12 et/ou du solide pulvérulent selon la revendication 9.

14. Médicament selon la revendication 13, **caractérisé en ce qu'**il est destiné au traitement du diabète.

15. Médicament selon la revendication 13 ou 14, **caractérisé en ce qu'**il est susceptible d'assurer au patient diabétique, après une injection sous-cutanée, un niveau basal d'insuline pendant au moins 24 heures..

16. Médicament selon l'une quelconque des revendications 13 à 15, **caractérisé en ce qu'**il est constitué par une présentation galénique comprenant, d'une part, du solide pulvérulent selon la revendication 9 et, d'autre part, de manière séparée, du liquide aqueux pour la reconstitution, avant l'administration, de la formulation selon l'une quelconque des revendications 1 à 8 et/ou de la formulation obtenue par le procédé selon l'une quelconque des revendications 10 à 12.

## Claims

1. Injectable long-acting insulin formulation comprising a stable aqueous colloidal suspension of insulin-laden nanoparticles of at least one poly(L-leucine-b-sodium L-glutamate) - hereafter called poly(Leu-block-Glu) - **characterized in that**:
I - the pH of the formulation is such that:
| | |
|---|---|
| | 5.8 ≤ pH ≤ 7.0 |
| preferably | 6.0 ≤ pH ≤ 7.0, |
II - the osmolarity O (in mOsmol) of the formulation is such that:
| | |
|---|---|
| | 270 ≤ O ≤ 800 |
| preferably | 270 ≤ O ≤ 600 |
| and particularly preferably | 270 ≤ O ≤ 400, |
III - the viscosity v (in mPa.s) of the formulation, measured at 20°C on an AR1000 rheometer (TA Instruments) equipped with a cone-and-plate geometry (4 cm, 2°) for a shear gradient of 10 s⁻¹, is low, namely such that:
| | |
|---|---|
| | v ≤ 40 |
| preferably | v ≤ 25 |
| and particularly preferably | v ≤ 20. |

2. Formulation according to claim 1, **characterized in that** its osmolarity O is adjusted by means of at least one monovalent or polyvalent salt.

3. Formulation according to claim 1 or 2, **characterized in that** the concentration of poly(Leu-block-Glu) is less than 60 mg/ml, preferably between 10 and 55 mg/ml and particularly preferably between 30 and 55 mg/ml.

4. Formulation according to one of the preceding claims, **characterized in that** the particles of chosen poly(Leu-block-Glu) have a mean hydrodynamic diameter Dh, expressed in nanometers (nm) and measured according to a procedure Md, such that:
| | |
|---|---|
| | 10 ≤ Dh ≤ 150 |
| preferably | 20 ≤ Dh ≤ 100, |
said procedure Md being as follows: the pulverulent powder of poly(Leu-block-Glu) is suspended in water at a concentration of about 50 g/l and the suspension is stirred overnight at 25°C. It is then diluted with 0.15 M aqueous sodium chloride solution to give a final poly(Leu-block-Glu) concentration comprised between 0.01 and 0.5 g/l and preferably of 0.2 g/l. This suspension is stirred for 1 hour and then introduced into the scattering cell of a Brookhaven light scattering apparatus operating with a vertically polarized laser beam of wavelength 488 nm.

5. Formulation according to one of the preceding claims, **characterized in that** the insulin/poly(Leu-block-Glu) weight ratio, expressed in %, is such that:
| | |
|---|---|
| | 3 ≤ insulin/poly(Leu-block-Glu), |
| preferably | 5 ≤ insulin/poly(Leu-block-Glu) ≤ 11. |

6. Formulation according to one of the preceding claims, **characterized in that** the maximum loading rate of the nanoparticles with insulin, Ta, expressed in % by weight of associated insulin relative to the weight of poly(Leu-block-Glu) and measured by a procedure Ma, is such that:
| | |
|---|---|
| | 10 ≤ Ta |
| preferably | 10 ≤ Ta ≤ 40 |
| and particularly preferably | 12 ≤ Ta ≤ 25, |
said procedure Md being as follows:
(a) Preparation of aqueous solutions of insulin: lyophilized recombinant human insulin is poured into a volume V of 0.01 N hydrochloric acid solution over at most 15 min; the resulting solution is then poured into the same volume V of 0.01 N NaOH solution; the pH is adjusted to between 7.2 and 7.4 with 1 N sodium hydroxide solution; the solution is then stirred gently for 30 min;
(b) Preparation of the insulin formulation: lyophilized poly(Leu-block-Glu) is added to the insulin solutions prepared at step (a) at a rate of 11 mg/ml; the resulting mixtures are degassed, then placed in a tilting stirrer at 25°C for 2 hours, and then degassed again; the pH is adjusted to between 7.2 and 7.4 with 1 N HCl solution; the resulting mixtures are stirred in a tilting stirrer overnight at room temperature;
(c) Assay of the free insulin: the formulations resulting from step (b) are injected onto a size exclusion liquid chromatography column under non-dissociating conditions and the free insulin is assayed by fluorimetry.

7. Formulation according to one of the preceding claims, **characterized in that** the insulin is an unmodified human insulin.

8. Formulation according to one of the preceding claims, **characterized in that** it comprises at least one preservative preferably selected from the group comprising phenols, cresols (e.g. metacresol), methyl, propyl or butyl parahydroxybenzoate and mixtures thereof.

9. Pulverulent solid, **characterized in that** it comprises insulin-laden nanoparticles of poly(Leu-block-Glu) and **in that** it is obtained from the formulation according to any one of claims 1 to 8.

10. Process for the preparation of the formulation according to any one of claims 1 to 8, **characterized in that** it consists essentially in mixing at least one poly(Leu-block-Glu) and insulin in an aqueous liquid medium, preferably with stirring, optionally adding excipients, adjusting the pH to a value of between 5.8 and 7.0 if necessary, and optionally filtering the resulting suspension.

11. Process for the preparation of the formulation according to any one of claims 1 to 8, **characterized in that** it consists essentially in mixing the poly(Leu-block-Glu) in the pulverulent state with an aqueous phase containing insulin.

12. Process for the preparation of the formulation according to any one of claims 1 to 8, **characterized in that** it consists essentially in suspending the pulverulent solid according to claim 8 in an aqueous liquid medium, preferably with stirring, optionally adding excipients, adjusting the pH to a value of between 5.8 and 7.0 if necessary, and optionally filtering the resulting suspension.

13. Drug, **characterized in that** it comprises an injectable long-acting insulin formulation according to any one of claims 1 to 8, and/or a formulation obtained by the process according to any one of claims 10 to 12, and/or a pulverulent solid according to claim 9.

14. Drug according to claim 13, **characterized in that** it is intended for the treatment of diabetes.

15. Drug according to claim 13 or 14, **characterized in that** it is capable of providing the diabetic patient with a basal insulin level for at least 24 hours after a subcutaneous injection.

16. Drug according to any one of claims 13 to 15, **characterized in that** it consists of a galenical presentation comprising on the one hand a pulverulent solid according to claim 9, and on the other hand, separately, an aqueous liquid with which the formulation according to any one of claims 1 to 8, and/or the formulation obtained by the process according to any one of claims 10 to 12, is reconstituted before administration.

## Patentansprüche

1. Injizierbare Insulinformulierung mit langer Wirkung, umfassend eine kolloidale, wässrige und stabile Lösung von Nanopartikeln von mindestens einem Poly(L-Leucin-b-L-Natriumglutamat) - im Folgenden als Poly(Leu-bloc-Glu) bezeichnet -, die mit Insulin beladen sind, **dadurch gekennzeichnet, dass**
I- der pH der Formulierung derart ist, dass:
| | |
|---|---|
| | 5,8 ≤ pH ≤ 7,0, |
| | 6,0 ≤ pH ≤ 7,0, |
II- die Osmolarität O (in mOsmol) der Formulierung derart ist, dass:
| | |
|---|---|
| | 270 ≤ O ≤ 800, |
| vorzugsweise | 270 ≤ O ≤ 600 |
| und noch stärker bevorzugt | 270 ≤ O ≤ 400, |
III- die Viskosität v (in mPa.s bei 20°C) der Formulierung, gemessen bei 20°C mithilfe eines AR1000-Rheometers (TA Instruments), das mit einer Kegel-Platte-Geometrie ausgestattet ist (4 cm, 2°), für einen Schergradienten von 10 s⁻¹ klein ist, nämlich derart, dass:
| | |
|---|---|
| | v ≤ 40, |
| vorzugsweise | v ≤ 25 |
| und noch stärker bevorzugt | v ≤ 20. |

2. Formulierung nach Anspruch 1, **dadurch gekennzeichnet, dass** ihre Osmolarität O mithilfe von mindestens einem einwertigen oder mehrwertigen Salz eingestellt wird.

3. Formulierung nach Anspruch 1 oder 2, **dadurch gekennzeichnet, dass** die Konzentration an Poly(Leu-bloc-Glu) kleiner als 60 mg/ml, vorzugsweise zwischen 10 und 55 mg/ml und noch stärker bevorzugt zwischen 30 und 55 mg/ml ist.

4. Formulierung nach einem der vorhergehenden Ansprüche, **dadurch gekennzeichnet, dass** die zurückgehaltenen Poly(Leu-bloc-Glu)-Partikel einen derartigen mittleren hydrodynamischen Durchmesser Dh, ausgedrückt in Nanometer (nm) und gemessen gemäß einer Durchführungsweise Md, haben, dass:
| | |
|---|---|
| | 10 ≤ Dh ≤ 150, |
| | 20 ≤ Dh ≤ 100, |
wobei die Durchführungsweise Md folgendermaßen ist: das pulverförmige Poly(Leu-bloc-Glu)-Pulver wird in Wasser in einer Konzentration von etwa 50 g/l in Suspension gebracht und über Nacht bei 25°C unter Rühren belassen. Es wird anschließend mithilfe einer wässrigen 0,15 M Natriumchloridlösung verdünnt, um eine Endkonzentration an Poly(Leu-bloc-Glu) zwischen 0,01 und 0,5 g/l, vorzugsweise gleich 0,2 g/l zu erhalten. Diese Suspension wird 1 Stunde lang gerührt, dann in die Diffusionszelle einer Lichtstreuungsapparatur des Brookhaven-Typs eingebracht, die mit einem vertikal polarisierten Laserbündel der Wellenlänge 488 nm arbeitet.

5. Formulierung nach einem der vorhergehenden Ansprüche, **dadurch gekennzeichnet, dass** das Massenverhältnis von Insulin/Poly(Leu-bloc-Glu), ausgedrückt in %, derart ist, dass:
| | |
|---|---|
| | 3 ≤ Insulin/Poly(Leu-bloc-Glu), |
| vorzugsweise | 5 ≤ Insulin/Poly(Leu-bloc-Glu) ≤ 11. |

6. Formulierung nach einem der vorhergehenden Ansprüche, **dadurch gekennzeichnet, dass** der maximale Beladungsgrad Ta der Nanopartikel mit Insulin, ausgedrückt in Massen-% an angelagertem Insulin, bezogen auf die Masse an Poly(Leu-bloc-Glu), gemessen gemäß einer Durchführungsweise Ma, derart ist, dass:
| | |
|---|---|
| | 10 ≤ Ta, |
| vorzugsweise | 10 ≤ Ta ≤ 40 |
| und noch stärker bevorzugt | 12 ≤ Ta ≤ 25, |
wobei die Durchführungsweise Ma folgendermaßen ist:
(a) Herstellung wässriger Insulinlösungen: lyophilisiertes rekombinantes humanes Insulin wird in ein Volumen V einer 0,01 N Salzsäurelösung für maximal 15 min eingebracht; die erhaltene Lösung wird anschließend in dasselbe Volumen V einer 0,01 N NaOH-Lösung überführt; der pH wird auf zwischen 7,2 und 7,4 mit einer 1 N Natronlaugelösung eingestellt; dann wird die Lösung für 30 min vorsichtig gerührt;
(b) Herstellung der Insulinformulierung: lyophilisiertes Poly(Leu-bloc-Glu) wird zu den unter (a) hergestellten Insulinlösungen zu 11 mg/ml hinzugefügt; die erhaltenen Gemische werden entgast und dann bei 25°C für 2 Stunden in einen Kippmischer platziert und anschließend erneut entgast; der pH wird mit einer 1 N HCl-Lösung auf zwischen 7,2 und 7,4 eingestellt; die erhaltenen Gemische werden in einem Kippmischer bei Umgebungstemperatur über Nacht gemischt;
(c) Messung des freien Insulins: die unter (b) erhaltenen Formulierungen werden auf eine Säule für die sterische Ausschluss-Flüssigkeitschromatographie unter nicht-dissoziierenden Bedingungen injiziert und das freie Insulin wird mittels Fluorimetrie bestimmt.

7. Formulierung nach einem der vorhergehenden Ansprüche, **dadurch gekennzeichnet, dass** es sich bei dem Insulin um unmodifiziertes humanes Insulin handelt.

8. Formulierung nach einem der vorhergehenden Ansprüche, **dadurch gekennzeichnet, dass** sie mindestens ein Konservierungsmittel enthält, das vorzugsweise aus der Gruppe ausgewählt ist, die Folgende umfasst: Phenole, Kresole (z. B. meta-Kresol), Methyl-, Propyl- oder Butylparahydroxybenzoate und deren Gemische.

9. Pulverförmiger Feststoff, **dadurch gekennzeichnet, dass** er mit Insulin beladene Poly(Leu-bloc-Glu)-Nanopartikel umfasst, und **dadurch**, dass er ausgehend von der Formulierung nach einem der Ansprüche 1 bis 8 erhalten wird.

10. Verfahren zur Herstellung der Formulierung nach einem der Ansprüche 1 bis 8, **dadurch gekennzeichnet, dass** man bei dem Verfahren im Wesentlichen mindestens ein Poly(Leu-bloc-Glu) und Insulin in einem wässrigen flüssigen Medium, vorzugsweise unter Rühren, mischt, gegebenenfalls Excipienten hinzufügt, nötigenfalls den pH auf einen Wert zwischen 5,8 und 7,0 einstellt und gegebenenfalls die so erhaltene Suspension filtriert.

11. Verfahren zur Herstellung der Formulierung nach einem der Ansprüche 1 bis 8, **dadurch gekennzeichnet, dass** man bei dem Verfahren im Wesentlichen das Poly(Leu-bloc-Glu) im pulverförmigen Zustand mit einer wässrigen Phase, die Insulin enthält, mischt.

12. Verfahren zur Herstellung der Formulierung nach einem der Ansprüche 1 bis 8, **dadurch gekennzeichnet, dass** man bei dem Verfahren im Wesentlichen den pulverförmigen Feststoff nach Anspruch 8 in einem flüssigen wässrigen Medium, vorzugsweise unter Rühren, in Suspension bringt, gegebenenfalls Excipienten hinzufügt, nötigenfalls den pH auf einen Wert zwischen 5,8 und 7,0 einstellt und gegebenenfalls die so erhaltene Suspension filtriert.

13. Arzneimittel, **dadurch gekennzeichnet, dass** es die injizierbare Insulinformulierung mit langer Wirkung nach einem der Ansprüche 1 bis 8 und/oder die Formulierung, die durch das Verfahren nach einem der Ansprüche 10 bis 12 erhalten wird, und/oder den pulverförmigen Feststoff nach Anspruch 9 umfasst.

14. Arzneimittel nach Anspruch 13, **dadurch gekennzeichnet, dass** es für die Behandlung von Diabetes bestimmt ist.

15. Arzneimittel nach Anspruch 13 oder 14, **dadurch gekennzeichnet, dass** es bei einem diabetischen Patienten nach einer subkutanen Injektion einen Basalspiegel an Insulin während mindestens 24 Stunden sicherstellen kann.

16. Arzneimittel nach einem der Ansprüche 13 bis 15, **dadurch gekennzeichnet, dass** es aus einer galenischen Darreichung besteht, die einerseits den pulverförmigen Feststoff nach Anspruch 9 und andererseits, in getrennter Form, wässrige Flüssigkeit für die Rekonstitution der Formulierung nach einem der Ansprüche 1 bis 8 und/oder der Formulierung, die durch das Verfahren nach einem der Ansprüche 10 bis 12 erhalten wird, vor der Verabreichung umfasst.
